(19) Europäisches Patentamt
European Patent Office
Office européen des brevets

(11) Veröffentlichungsnummer: **0 328 954**
**A1**

(12) # EUROPÄISCHE PATENTANMELDUNG

(21) Anmeldenummer: **89101843.4**

(51) Int. Cl.⁴: **C07D 277/56 , A01N 43/78**

(22) Anmeldetag: **03.02.89**

(30) Priorität: **13.02.88 DE 3804531**

(43) Veröffentlichungstag der Anmeldung: **23.08.89 Patentblatt 89/34**

(84) Benannte Vertragsstaaten: **BE CH DE ES FR GB IT LI NL**

(71) Anmelder: **BAYER AG**

**D-5090 Leverkusen 1 Bayerwerk(DE)**

(72) Erfinder: **Kluth, Joachim, Dr.**
**Kurt-Schumacher-Strasse 9**
**D-4018 Langenfeld(DE)**
Erfinder: **Tietjen, Klaus-Günther, Dr.**
**Am Alten Broich 64a**
**D-4018 Langenfeld(DE)**
Erfinder: **Brandes, Wilhelm, Dr.**
**Eichendorffstrasse 3**
**D-5653 Leichlingen(DE)**
Erfinder: **Santel, Hans-Joachim, Dr.**
**Grünstrasse 9a**
**D-5090 Leverkusen 1(DE)**
Erfinder: **Schmidt, Robert, R., Dr.**
**Im Waldwinkel 110**
**D-5060 Bergisch-Gladbach 2(DE)**
Erfinder: **Strang, Harry, Dr.**
**Unterdorfstrasse 6A**
**D-4000 Düsseldorf 31(DE)**

(54) **5-Cyano-2,4-diamino-thiazol-Derivate.**

(57) Die vorliegende Erfindung betrifft 5-Cyano-2,4-diaminothiazol-Derivate der allgemeinen Formel (I)

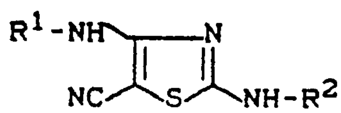

(I)

in welcher
$R^1$ für Wasserstoff oder für einen gegebenenfalls substituierten Rest aus der Reihe Alkyl, Alkenyl, Alkinyl steht oder für die Gruppierung -CO-$R^3$ steht, worin,
$R^3$ für einen gegebenenfalls substituierten Rest aus der Reihe Alkyl, Alkoxy, Alkylthio, Alkylamino und Dialkylamino steht, und
$R^2$ für einen gegebenenfalls substituierten Rest aus der Reihe Alkyl, Alkenyl, Cycloalkyl, Aryl, Aralkyl, Heteroaryl und Heteroarylalkyl steht,
ein Verfahren zu deren Herstellung und ihre Verwendung als Herbizide und Fungizide.

EP 0 328 954 A1

Xerox Copy Centre

# 5-Cyano-2,4-diamino-thiazol-Derivate

Die vorliegende Erfindung betrifft neue 5-Cyano-2,4-diamino-thiazol-Derivate, ein Verfahren zu ihrer Herstellung und ihre Verwendung als Herbizide und Fungizide.

Es ist bekannt, daß bestimmte Arylaminoverbindungen, wie z. B. N-(3-Methyl-phenyl)-O-(3-methoxycarbonylaminophenyl)-carbaminsäureester (Phenmedipham, Betanal) herbizid wirksam sind (vgl. DE-AS 15 67 151).

Weiter ist bekannt, daß bestimmte organische Cyanoverbindungen, wie z. B. 2-Cyano-N-(ethylaminocarbonyl)-2-methoxyimino-acetamid (Cymoxanil, Curzate) fungizide Eigenschaften aufweisen (vgl. US-P 3 957 847).

Bestimmte 5-Cyano-2,4-diamino-thiazol-Derivate, und zwar 2-Allylamino-4-amino-5-cyano-thiazol und 2-Phenylamino-4-amino-5-cyano-thiazol, sind bereits aus der Literatur bekannt (vgl. Monatshefte für Chemie 112 (1981), 1393-1404). Angaben zu physiologischen Eigenschaften dieser Verbindungen werden dort jedoch nicht gemacht.

Es wurden nun neue 5-Cyano-2,4-diamino-thiazol-Derivate der allgemeinen Formel (I)

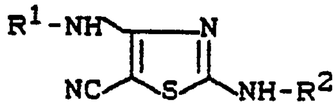

(I)

in welcher

$R^1$ für Wasserstoff oder für einen gegebenenfalls substituierten Rest aus der Reihe Alkyl, Alkenyl oder Alkinyl steht oder für die Gruppierung $-CO-R^3$ steht, worin,

$R^3$ für einen gegebenenfalls substituierten Rest aus der Reihe Alkyl, Alkoxy, Alkylthio, Alkylamino und Dialkylamino steht, und

$R^2$ für einen gegebenenfalls substituierten Rest aus der Reihe Alkyl, Alkenyl, Cycloalkyl, Aryl, Aralkyl, Heteroaryl und Heteroarylalkyl steht,

gefunden, wobei 2-Allylamino-4-amino-5-cyano-thiazol und 2-Phenylamino-4-amino-5-cyano-thiazol ausgenommen sind.

Weiter wurde gefunden, daß man die neuen 5-Cyano-2,4-diamino-thiazol-Derivate der allgemeinen Formel (I) er hält, wenn man Isothiocyanate der allgemeinen Formel (II)

$R^2 - N = C = S$ (II)

in welcher

$R^2$ die oben angegebene Bedeutung hat,

mit Cyanamid in Gegenwart eines Alkalimetallhydroxides oder eines Alkalimetallalkoholates bzw. mit einem Alkalimetallsalz von Cyanamid

und anschließend mit Bromacetonitril oder Chloracetonitril

gegebenenfalls in Gegenwart eines Verdünnungsmittels umsetzt und die so erhaltenen Verbindungen der Formel (I), in welcher dann $R^1$ für Wasserstoff steht - gegebenenfalls nach Zwischenisolierung - gegebenenfalls mit Verbindungen der Formel (III)

$R^1 - X$ (III)

in welcher

$R^1$ mit Ausnahme von Wasserstoff die oben angegebene Bedeutung hat und

X für Halogen steht,

gegebenenfalls in Gegenwart eines Säureakzeptors und gegebenenfalls in Gegenwart eines Verdünnungsmittels umsetzt.

Schließlich wurde gefunden, daß die neuen 5-Cyano-2,4-diamino-thiazol-Derivate der allgemeinen Formel (I) starke herbizide und fungizide Eigenschaften aufweisen. Ebenso zeigen die bekannten 5-Cyano-2,4-diamino-thiazol-Derivate - welche bei der Definition der Verbindungen der Formel (I) durch einen "Disclaimer" ausgenommen sind - fungizide und herbizide Eigenschaften.

Die Erfindung betrifft also die Verwendung der erfindungsgemäßen Verbindungen der Formel (I) und der bekannten 5-Cyano-2,4-diamino-thiazol-Derivate, welche in der folgenden Formel (Ia) zusammengefaßt sind

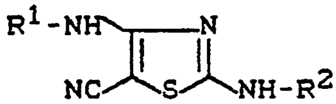

(Ia)

in welcher
$R^1$ für Wasserstoff oder für einen gegebenenfalls substituierten Rest aus der Reihe Alkyl, Alkenyl oder Alkinyl steht oder für die Gruppierung -CO-$R^3$ steht, worin,
$R^3$ für einen gegebenenfalls substituierten Rest aus der Reihe Alkyl, Alkoxy, Alkylthio, Alkylamino und Dialkylamino steht, und
$R^2$ für einen gegebenenfalls substituierten Rest aus der Reihe Alkyl, Alkenyl, Cycloalkyl, Aryl, Aralkyl, Heteroaryl und Heteroarylalkyl steht.

Überraschenderweise zeigen die erfindungsgemäßen 5-Cyano-2,4-diamino-thiazol-Derivate der allgemeinen Formel (I) bzw. (Ia) eine erheblich stärkere Wirkung gegen Unkräuter als der bekanne N-(3-Methylphenyl-O-(3-methoxycarbonylamino-phenyl)-carbaminsäureester, welcher als Herbizid im Handel ist, und sind auch gegen pilzliche Pflanzenkrankheiten besser wirksam als 2-Cyano-N-(ethylamino-carbonyl)-2-methoximino-acetamid, welches ein bekanntes Fungizid ist.

Sofern die 5-Cyano-2,4-diamino-thiazol-Derivate der Formel (I) bzw. (Ia) ein asymmetrisches Kohlenstoffatom besitzen, betrifft die Erfindung sowohl die Racemate als auch die einzelnen optisch aktiven Isomere, wobei von den optisch aktiven Isomeren die S-Enantiomere bevorzugt sind.

Die Kohlenstoffketten in den Resten wie beispielsweise Alkyl, Alkoxy, Alkenyl, Alkoxycarbonyl, Halogenalkyl, Alkylsulfonyl sind jeweils geradkettig oder verzweigt.

Die Erfindung betrifft vorzugsweise Verbindungen der Formel (I), in welcher
$R^1$ für Wasserstoff, für gegebenenfalls durch Halogen, Cyano, $C_1$-$C_4$-Alkoxy oder $C_1$-$C_4$-Alkoxy-carbonyl substituiertes $C_1$-$C_6$-Alkyl, für gegebenenfalls durch Halogen, Cyano oder $C_1$-$C_4$-Alkoxy-carbonyl substituiertes $C_2$-$C_6$-Alkenyl, für gegebenenfalls durch Halogen, Cyano oder $C_1$-$C_4$-Alkoxy-carbonyl substituier tes $C_2$-$C_6$-Alkinyl oder für die Gruppierung -CO-$R^3$ steht, worin
$R^3$ für einen gegebenenfalls durch Halogen, Cyano, $C_1$-$C_4$-Alkoxy, $C_3$-$C_6$-Cycloalkyl, $C_1$-$C_4$-Alkoxy-carbonyl oder Phenyl substituierten Rest aus der Reihe $C_1$-$C_6$-Alkyl, $C_1$-$C_6$-Alkoxy, $C_1$-$C_6$-Alkylthio, $C_1$-$C_6$-Alkylamino und Di-($C_1$-$C_4$-alkyl)-amino steht, und
$R^2$ für gegebenenfalls durch Halogen, Cyano, $C_1$-$C_4$-Alkoxy, $C_3$-$C_6$-Cycloalkyl oder $C_1$-$C_4$-Alkoxy-carbonyl substituiertes $C_1$-$C_6$-Alkyl, für $C_3$-$C_6$-Cycloalkyl, für gegebenenfalls durch Halogen, Cyano oder $C_1$-$C_4$-Alkoxy-carbonyl substituiertes $C_2$-$C_6$-Alkenyl, für gegebenenfalls durch Halogen, Cyano, Nitro, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkylsulfinyl, $C_1$-$C_2$-Halogenalkylsulfinyl, $C_1$-$C_4$-Alkylsulfonyl, $C_1$-$C_2$-Halogenalkylsulfonyl, $C_1$-$C_2$-Alkylendioxy und/oder $C_1$-$C_4$-Alkoxy-carbonyl substituierte Reste aus der Reihe Phenyl, Naphthyl, Pyridyl, Chinolinyl, Isochinolinyl, Pyrimidinyl, Furyl oder Thienyl steht, oder für gegebenenfalls durch Halogen, Cyano, Nitro, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkylsulfinyl, $C_1$-$C_2$-Halogenalkylsulfinyl, $C_1$-$C_4$-Alkylsulfonyl, $C_1$-$C_2$-Halogenalkylsulfonyl und/oder $C_1$-$C_2$-Alkylendioxy substituierte Reste aus der Reihe Phenyl-$C_1$-$C_4$-alkyl, Naphthyl- $C_1$-$C_4$-alkyl, Pyridyl-$C_1$-$C_4$-alkyl, Pyrimidinyl-$C_1$-$C_4$-alkyl, Furyl-$C_1$-$C_4$-alkyl oder Thienyl-$C_1$-$C_4$-alkyl steht,
wobei 2-Allylamino-4-amino-5-cyano-thiazol und 2-Phenylamino-4-amino-5-cyano-thiazol ausgenommen sind.

Die Erfindung betrifft insbesondere Verbindungen der Formel (I), in welcher
$R^1$ für Wasserstoff, $C_1$-$C_4$-Alkyl, $C_3$-$C_4$-Alkenyl, $C_3$-$C_4$-Alkinyl oder für die Gruppierung -CO-$R^3$ steht, worin
$R^3$ für gegebenenfalls durch Fluor, Chlor und/oder Brom substituiertes $C_1$-$C_4$-Alkyl, $C_1$-$C_2$-Alkoxy-$C_1$-$C_2$-alkyl, $C_1$-$C_4$-Alkoxy oder $C_1$-$C_4$-Alkylthio steht, und
$R^2$ für $C_1$-$C_5$-Alkyl, für Cyclohexyl, für durch Fluor, Chlor, Brom, Methyl, Ethyl, Trifluormethyl, Methoxy, Ethoxy, Difluormethoxy, Trifluormethoxy, Methylthio, Ethylthio und/oder Trifluormethylthio substituiertes Phenyl, für gegebenenfalls durch Fluor, Chlor, Brom, Methyl, Ethyl, Trifluormethyl, Methoxy, Ethoxy, Difluormethoxy, Trifluormethoxy und/oder Methylendioxy substituiertes Phenyl-$C_1$-$C_3$-alkyl, für Naphthyl-$C_1$-$C_3$-alkyl oder für gegebenenfalls durch Fluor, Chlor, Brom, Methyl, Ethyl, Trifluormethyl, Methoxy und/oder Ethoxy substituiertes Pyridinyl-$C_1$-$C_3$-alkyl steht.

Ganz besonders bevorzugt werden diejenigen Verbindungen der Formel (I), in welcher
$R^1$ für die Gruppierung -CO-$R^3$ steht, worin
$R^3$ für Ethyl, Propyl, Ethoxymethyl, Methoxymethyl oder 2-Methoxy-ethyl steht, und
$R^2$ für Propyl, Isopropyl, Butyl, Isobutyl, sec-Butyl oder tert-Butyl steht oder für Benzyl oder 1-Phenyl-ethyl

steht, welche in der Phenylkomponente gegebenenfalls durch Fluor, Chlor, Brom, Methyl und/oder Methoxy substituiert sind.

Die Substituenten $R^1$ und $R^2$ der Formel (Ia) besitzen bevorzugt bzw. besonders bevorzugt bzw. ganz besonders bevorzugt diejenigen Bedeutungen, die bei der Beschreibung der Verbindungen der Formel (I) für diese Reste als bevorzugt bzw. besonders bevorzugt bzw. ganz besonders bevorzugt genannt wurden.

Beispiele für die erfindungsgemäßen Verbindungen der Formel (I) - bzw. (Ia) - sind in der nachstehenden Tabelle 1 aufgeführt - vgl. auch die Herstellungsbeispiele.

**Tabelle 1: Beispiele für die Verbindungen der Formel (I)**

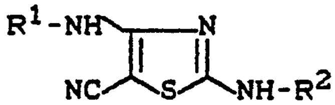

(I)

| $R^1$ | $R^2$ |
|---|---|
| H | $CH_3$ |
| H | $C_2H_5$ |
| H | $C_3H_7$ |
| H | $CH(CH_3)_2$ |
| H | $C_4H_9$ |
| H | $CH_2CH(CH_3)_2$ |
| H | $CH(CH_3)CH_2CH_3$ |
| H | $C(CH_3)_3$ |
| H | 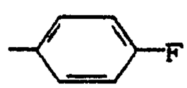 |
| H | 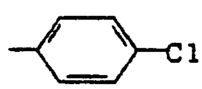 |
| H | 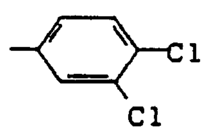 |
| H | 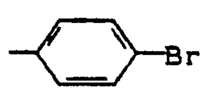 |
| H | 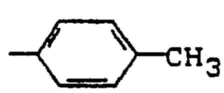 |

<u>Tabelle 1</u> - Fortsetzung

| $R^1$ | $R^2$ |
|---|---|
| H | 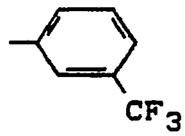 |
| H | 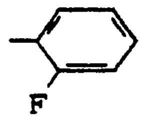 |
| H | 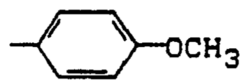 |
| H | 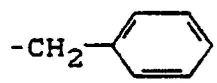 |
| H | 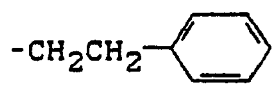 |
| H | 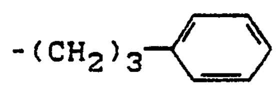 |
| H | 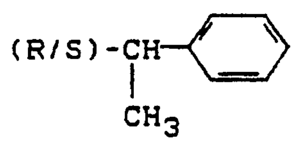 |
| H | 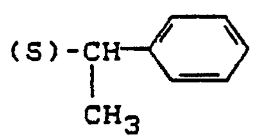 |
| H | 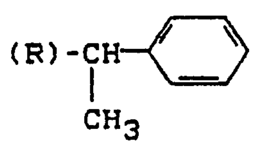 |
| H | 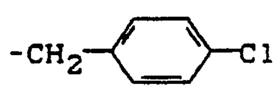 |

Tabelle 1 - Fortsetzung

| $R^1$ | $R^2$ |
|---|---|
| H | 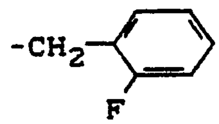 |
| H | 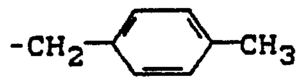 |
| H | 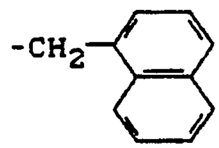 |
| H | 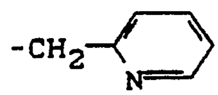 |
| H | 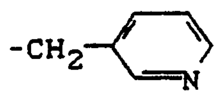 |
| H | 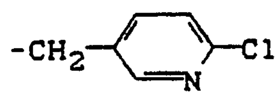 |
| H | 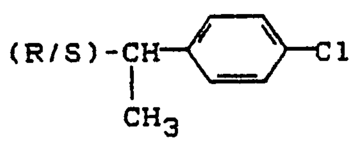 |
| H | 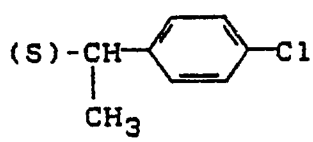 |
| $-CO-CH_3$ | $C_3H_7$ |
| $-CO-C_2H_5$ | $C_2H_5$ |
| $-CO-CH_3$ | $CH(CH_3)_2$ |
| $-CO-CH_3$ | $CH_2CH(CH_3)_2$ |

<u>Tabelle 1</u> - Fortsetzung

| $R^1$ | $R^2$ |
|---|---|
| $-CO-CH_3$ | $C(CH_3)_3$ |
| $-CO-C_2H_5$ | $CH_3$ |
| $-CO-CH_3$ | 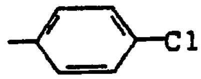 |
| $-CO-C_2H_5$ | 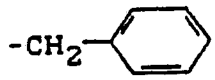 |
| $-CO-C_3H_7$ | 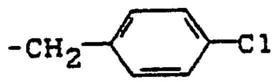 |
| $-CO-OCH_3$ | 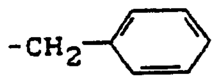 |
| $-CO-SC_2H_5$ | 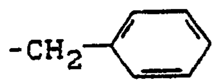 |
| $-CO-CH_2OC_2H_5$ | 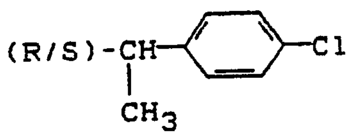 |
| $-CO-CH_2CH_2OCH_3$ | 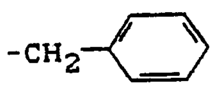 |
| $-CO-C_2H_5$ | 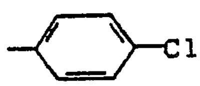 |
| $-CO-CH_3$ | 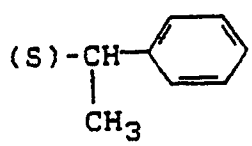 |

Tabelle 1 - Fortsetzung

| $R^1$ | $R^2$ |
|---|---|
| $-CO-C_2H_5$ | (S)- 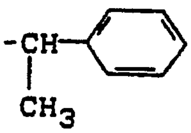 |
| $-CO-CH_3$ | (S)- 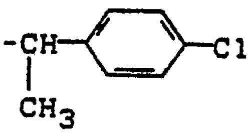 |
| $-CO-C_2H_5$ | (S)- 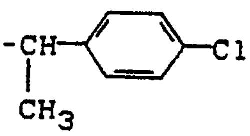 |
| $-CO-C_2H_5$ | $-C_4H_9$ |
| $-CO-C_2H_5$ | $-CH_2CH(CH_3)_2$ |
| $-CO-C_2H_5$ | (R/S)- 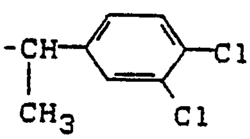 |
| $-CO-C_2H_5$ | (S)- 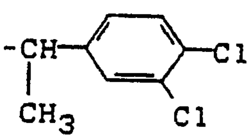 |
| $-CO-C_2H_5$ | (R/S)- 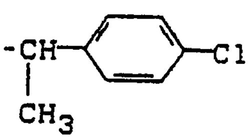 |
| $-CO-C_2H_5$ | (R/S)- 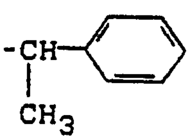 |
| $-CO-C_2H_5$ | (R/S)- 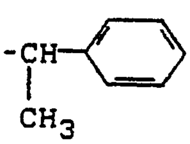 |

Tabelle 1 - Fortsetzung

| $R^1$ | $R^2$ |
|---|---|
| $-CO-C_2H_5$ | 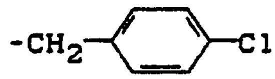 |
| $-CO-C_2H_5$ | 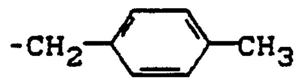 |
| $-CO-C_2H_5$ | 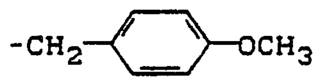 |
| $-CO-C_2H_5$ | 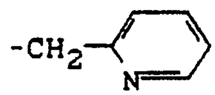 |
| $-CO-C_2H_5$ | 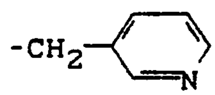 |
| $-CO-C_2H_5$ | 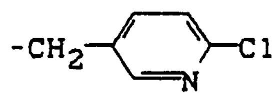 |
| $-CO-CH_2Cl$ | 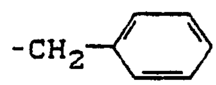 |
| $-CO-CHCl_2$ | 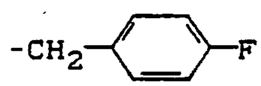 |
| $-CO-CF_3$ | 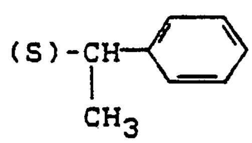 |
| $-CO-CH_2CH_2Cl$ | 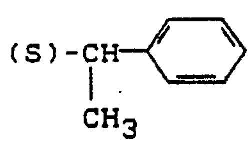 |

Tabelle 1 - Fortsetzung

| $R^1$ | $R^2$ |
|---|---|
| $-CO-CH_2OCH_3$ | 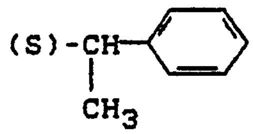 |
| $-CO-CH_3$ | 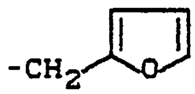 |
| H | 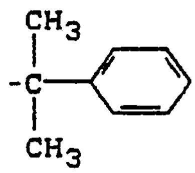 |
| $-CO-C_2H_5$ | 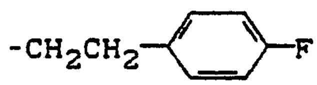 |
| 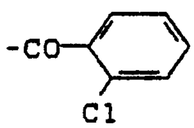 | 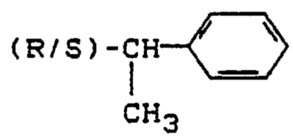 |

Verwendet man für das erfindungsgemäße Verfahren neben Cyanamid beispielsweise Benzylisothiocyanat und Kalium-tert-butanolat, anschließend Chloracetonitril und schließlich Chlorameisensäure-methylester als Ausgangsstoffe, so kann der Verlauf des erfindungsgemäßen Verfahrens durch das folgende Formelschema wiedergegeben werden:

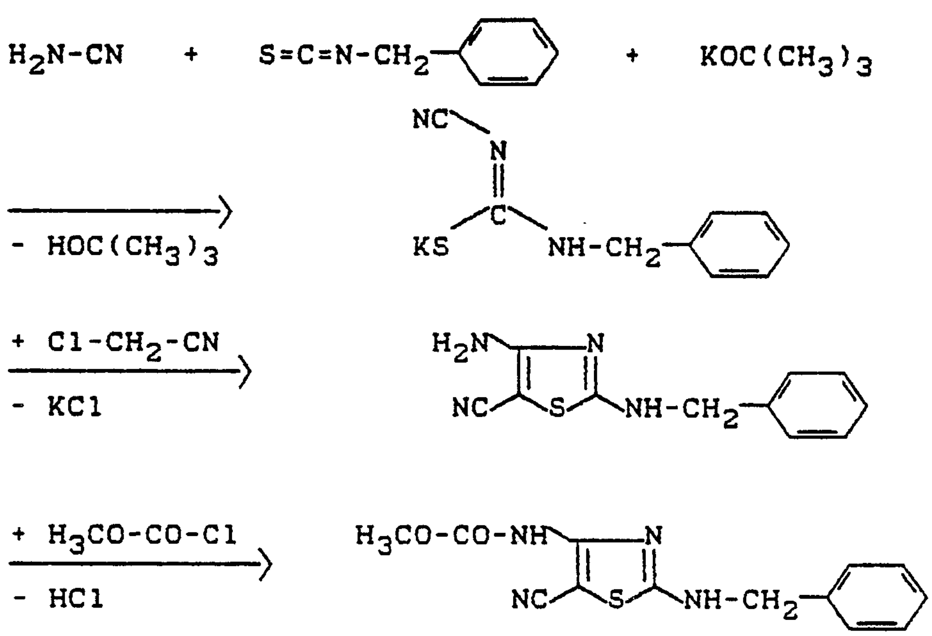

Die beim erfindungsgemäßen Verfahren als Ausgangsstoffe benötigten Isothiocyanate sind durch die Formel (II) allgemein definiert. In Formel (II) hat $R^2$ vorzugsweise bzw. insbesondere bzw. ganz besonders bevorzugt die gleiche Bedeutung, wie sie bereits im Zusammenhang mit der Beschreibung der erfindungsgemäßen Verbindungen der Formel (I) vorzugsweise bzw. insbesondere bzw. ganz besonders bevorzugt für $R^2$ angegeben ist.

Als Beispiele für die Ausgangsstoffe der Formel (II) seien genannt:
Methyl-, Ethyl-, Propyl-, Isopropyl-, Butyl-, Isobutyl-, sec-Butyl-, tert-Butyl-, 4-Fluor-phenyl-, 2-Fluor-phenyl-, 4-Chlor-phenyl, 3,4-Dichlor-phenyl-, 3,5-Dichlor-phenyl-, 4-Brom-phenyl-, 3-Methyl-phenyl-, 4-Methyl-phenyl, 3-Trifluormethyl-phenyl-, 4-Trifluormethyl-phenyl-, 4-Methoxy-phenyl-, 4-Difluormethoxy-phenyl-, 4-Trifluormethoxy-phenyl-, 4-Ethoxy-phenyl-, 4-Methylthio-phenyl-, 4-Trifluormethylthio-phenyl-, 4-Chlordifluormethylthio-phenyl-, Benzyl-, 4-Chlor-benzyl, 4-Fluor-benzyl, 2-Fluor-benzyl-, 4-Methyl-benzyl, 4-Methoxy-benzyl, 2-Phenyl-ethyl-, 3-Phenyl-ethyl-, (R/S)-1-Phenyl-ethyl-, (S)-1-Phenyl-ethyl-, (R)-1-Phenyl-ethyl-, (R/S)-1-(4-Chlor-phenyl)-ethyl-, (S)-1-(4-Chlor-phenyl)-ethyl-, (R/S)-1-(3,4-Dichlor-phenyl)-ethyl-, (S)-1-(3,4-Dichlor-phenyl)-ethyl-, 2-Phenyl-propyl-, 1-Phenyl-propyl-, Pyridin-2-yl-methyl-, Pyridin-3-yl-methyl-, 2-Chlor-pyridin-5-yl-methyl-, Furan-2-yl-methyl- und Thiophen-2-yl-methyl-isothiocyanat.

Die Ausgangsstoffe der Formel (II) sind bekannt und/oder können nach an sich bekannten Verfahren hergestellt werden (vgl. Houben-Weyl, Methoden der organischen Chemie, Band 9 (1955), S. 867 - 878 und Band E4 (1983), S. 834 -869.

Als weiterer Ausgangsstoff wird beim erfindungsgemäßen Verfahren Cyanamid in Gegenwart eines Alkalimetallhydroxids oder eines Alkalimetall-alkoholats bzw. ein Alkalimetallsalz von Cyanamid verwendet.

Als Beispiele für geeignete Alkalimetall-hydroxide bzw. Alkalimetall-alkoholate seien genannt:
Lithium-hydroxid, Natrium-hydroxid, -methylat, -ethylat, -propylat, -isopropylat, -butylat, -isobutylat, sec-butylat und -tert-butylat sowie Kalium-hydroxid, -methylat, -ethylat, -propylat, -isopropylat, -butylat, -isobutylat, -sec-butylat und -tert-butylat.
Vorzugsweise wird Kalium-tert-butylat verwendet.

Als Beispiele für Alkalimetallsalze von Cyanamid seien Natrium-cyanamid und Kalium-cyanamid genannt.

Die vorausgehend genannten Ausgangsstoffe sind - ebenso wie das in der Folge zu verwendende Chloracetonitril bzw. Bromacetonitril - bekannte Synthesechemikalien.

Das erfindungsgemäße Verfahren wird gegebenenfalls unter Einsatz von Verbindungen der allgemeinen Formel (III) durchgeführt. In Formel (III) hat $R^1$ mit Ausnahme von Wasserstoff vorzugsweise bzw. insbesondere bzw. ganz besonders bevorzugt die gleiche Bedeutung, wie sie bereits im Zusammenhang mit der Beschreibung der erfindungsgemäßen Verbindungen der Formel (I) vorzugsweise bzw. insbesondere bzw. ganz besonders bevorzugt für $R^1$ angegeben ist.

Als Beispiele für die Ausgangsstoffe der Formel (III) seien genannt:
Methyl-, Ethyl-, Propyl-, Isopropyl-, Butyl-, Isobutyl-und sec-Butyl-chlorid, -bromid und -iodid, Acetyl-, Propionyl-, Butyroyl-, Isobutyroyl-, Chloracetyl-, Dichlor acetyl-, Trichloracetyl-, 2-Chlor-propionyl-, 2-Brom-propionyl-, Methoxyacetyl-, Ethoxyacetyl-, 2-Methoxy-propionyl- und 2-Ethoxy-propionyl-chlorid, Chlorameisensäure-methylester, -ethylester, -propylester und butylester sowie Chlorthioameisensäure-S-methylester, -S-ethylester, -S-propylester und -S-butylester.

Die Ausgangsstoffe der Formel (III) sind bekannte Synthesechemikalien.

Das erfindungsgemäße Verfahren zur Herstellung der neuen Verbindungen der Formel (I) bzw. (Ia) wird vorzugsweise unter Verwendung von Verdünnungsmitteln durchgeführt. Als Verdünnungsmittel kommen dabei praktisch alle inerten organischen Lösungsmittel in Frage. Hierzu gehören vorzugsweise aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Diethyl- und Dibutylether, Glykoldimethylether und Diglykoldimethylether, Tetrahydrofuran und Dioxan, Ketone wie Aceton, Methyl-ethyl-, Methyl-isopropyl- und Methyl-isobutyl-keton, Ester wie Essigsäuremethylester und -ethylester, Nitrile wie z. B. Acetonitril und Propionitril, Amide wie z. B. Dimethylformamid, Dimethylacetamid und N-Methyl-pyrrolidon sowie Dimethylsulfoxid, Tetramethylensulfon und Hexamethylphosphorsäuretriamid.

Es können auch die den oben genannten Alkalimetallalkoholaten entsprechenden Alkohole als Verdünnungsmittel verwendet werden.

Die gegebenenfalls beim erfindungsgemäßen Verfahren durchzuführende Umsetzung der Verbindungen der Formel (I), in welcher $R^1$ für Wasserstoff steht, mit Verbindungen der Formel (III) wird gegebenenfalls in Gegenwart eines Säureakzeptors durchgeführt.

Als Säureakzeptoren können bei dem erfindungsgemäßen Verfahren alle üblicherweise für derartige Umsetzungen verwendbaren Säurebindemittel eingesetzt werden. Vorzugsweise in Frage kommen Alkalimetallhydroxide wie z. B. Natrium- und Kaliumhydroxid, Erdalkalihydroxide wie z. B. Calciumhydroxid, Alkalicarbonate und -alkoholate wie Natrium- und Kaliumcarbonat, Natrium- und Kalium-tert-butylat, ferner aliphatische, aromatische oder heterocyclische Amine, beispielsweise Triethylamin, Trimethylamin, Dimethylanilin, Dimethylbenzylamin, Pyridin, 1,5-Diazabicyclo-[4,3,0]-non-5-en (DBN), 1,8-Diazabicyclo-[5,4,0]-undec-7-en (DBU) und 1,4-Diazabicyclo-[2,2,2]-octan (DABCO).

Die Reaktionstemperaturen können bei dem erfindungsgemäßen Verfahren in einem größeren Bereich variiert werden. Im allgemeinen arbeitet man bei Temperaturen zwischen -20 °C und +100 °C, vorzugsweise bei Temperaturen zwischen -10 °C und +50 °C.

Das erfindungsgemäße Verfahren wird im allgemeinen unter Normaldruck durchgeführt. Es ist jedoch auch möglich, unter erhöhtem oder vermindertem Druck zu arbeiten.

Zur Durchführung des erfindungsgemäßen Verfahrens werden die Ausgangsstoffe im allgemeinen in annähernd äquimolaren Mengen eingesetzt. Es ist jedoch auch möglich, einzelne Komponenten in mehr oder weniger großen Überschüssen zu verwenden.

In einer bevorzugten Ausführungsform des erfindungsgemäßen Verfahrens wird ein Alkalimetallsalz von Cyanamid - welches man gegebenenfalls aus Cyanamid und einem Alkalimetall-hydroxid oder -alkoholat in einem der oben angegebenen Verdünnungsmittel erzeugt hat und vorzugsweise in situ einsetzt - vorgelegt und unter Kühlen und Rühren langsam mit einem Isothiocyanat der Formel (II) versetzt. Nach kurzem Rühren wird dann Brom- oder Chloracetonitril zum Reaktionsgemisch gegeben und die komplette Mischung bis zum Ende der Umsetzung gerührt. Das Produkt dieser Umsetzung (Verbindungen der Formel (I) mit $R^1$ = H) kann auf übliche Weise isoliert werden. Es kann jedoch auch ohne Zwischenisolierung durch Zugabe einer Verbindung der Formel (III) - und vorzugsweise eines der oben angegebenen Säureakzeptoren - zu einem Produkt der Formel (I), bei dem $R^1$ von Wasserstoff verschieden ist, umgesetzt werden.

Zur Aufarbeitung wird im allgemeinen - gegebenenfalls nach Einengen - mit Wasser und einem mit Wasser praktisch nicht mischbaren organischen Lösungsmittel, wie z. B. Methylenchlorid, geschüttelt, gegebenenfalls der Rest Säureakzeptor durch Waschen mit einer wäßrigen Säure, wie z. B. Salzsäure, entfernt, die organische Phase mit einem üblichen Trockenmittel, wie z. B. Natriumsulfat getrocknet und filtriert. Vom Filtrat wird das Lösungsmittel unter vermindertem Druck sorgfältig abdestilliert. Der verbleibende Rückstand enthält im wesentlichen das Produkt der Formel (I).

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden pflanzen verwendet werden:

<u>Dikotyle Unkräuter der Gattungen:</u>

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea.

<u>Dikotyle Kulturen der Gattungen:</u>

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

<u>Monokotyle Unkräuter der Gattungen:</u>

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

<u>Monokotyle Kulturen der Gattungen:</u>

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Die erfindungsgemäßen Wirkstoffe eignen sich vor allem zur selektiven Bekämpfung dikotyler Unkräuter in monokotylen Kulturen, insbesondere im Nachauflauf-Verfahren.

Die erfindungsgemäßen Wirkstoffe weisen auch eine starke fungizide Wirkung auf. Sie sind insbesondere zur Bekämpfung von Schadpilzen aus der Klasse der Oomyceten, wie z. B. Phytophthora infestans, geeignet.

Fungizide Mittel im Pflanzenschutz werden eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen Erkrankungen, die unter die oben aufgezählten Oberbegriffe fallen, genannte:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubensis;
Plasmopara-Arten, wie beispielsweise Plasmopara viticola;
Peronospora-Arten, wie beispielsweise Peronospora pisi oder P. brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielsweise Pyrenophora teres oder P. graminea
(Konidienform: Drechslera, Syn: Helminthosporium);
Cochliobolus-Arten, wie beispielsweise Cochliobolus sativus
(Konidienform: Drechslera, Syn: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsion-Konzentrate, Wirkstoff-imprägnierte Natur- und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol

oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N′-dimethyl-harnstoff (METABENZTHIAZURON) zur Unkrautbekämpfung in Getreide; 4-Amino-3-methyl-6-phenyl-1,2,4-triazin-5-(4H)-on (METAMITRON) zur Unkrautbekämpfung in Zuckerrüben und 4-Amino-6-(1,1,-dimethylethyl)-3-methylthio-1,2,4-triazin-5(4H)-on (METRIBUZIN) zur Unkrautbekämpfung in Sojabohnen, in Frage, ferner auch 2,4-Dichlorphenoxyessigsäure (2,4-D); 4-(2,4-Dichlorphenoxy)-buttersäure (2,4-DB); 2,4-Dichlorphenoxypropionsäure (2,4-DP); 3-Isopropyl-2,1,3-benzothiadiazin-4-on-2,2-dioxid (BENTAZON); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N′-(3-chlor-4-methylphenyl)-harnstoff (CHLORTOLURON); 2-[4-(2,4-Dichlorphenoxy)-phenoxy]-propionsäure, deren Methyl- oder deren Ethylester (DICLOFOP); 4-Amino-6-t-butyl-3-ethylthio-1,2,4-triazin-5-(4H)-on (ETHIOZIN); 2-{4-[(6-Chlor-2-benzoxazolyl)-oxy]-phenoxy}-propansäure, deren Methyl- oder deren Ethylester (FENOXAPROP); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N′-(4-isopropylphenyl)-harnstoff (ISOPROTURON); (2-Methyl-4-chlorphenoxy)-essigsäure (MCPA); 4-(4-Chlor-2-methyl)-phenoxybuttersäure (MCPB); (4-Chlor-2-methylphenoxy)-propionsäure (MCPP); N-Methyl-2-(1,3-benzthiazol-2-yloxy)-acetanilid (MEFENACET); 2-{[[((4-Methoxy-6-methyl-1,3,5-triazin-2-yl)-amino)-carbonyl]-amino]-sulfonyl}-benzoesäure oder deren Methylester (METASULFURON); N-(1-Ethylpropyl)-3,4-dimethyl-2,6-dinitroanilin (PENDIMETHALIN); O-(6-Chlor-3-phenyl-pyridazin-4-yl)-S-octyl-thiocarbonat (PYRIDATE); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE) und 3,5,6-Trichlor-2-pyridyloxyessigsäure (TRICLOPYR). Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Bei der Anwendung als Herbizid kann die angewandte Wirkstoffmenge in einem größeren Bereich schwanken. Sie hängt im wesentlichen von der Art des gewünschten Effektes ab. Im allgemeinen liegen die Aufwandmengen zwischen 0,01 und 15 kg Wirkstoff pro Hektar Bodenfläche, vorzugsweise zwischen 0,05 und 10 kg pro ha.

Bei der Behandlung von Pflanzenteilen (Anwendung als Fungizid) können die Wirkstoffkonzentrationen in den Anwendungsformen in einem größeren Bereich variiert werden. Sie liegen im allgemeinen zwischen 1 und 0,0001 Gew.-%, vorzugsweise zwischen 0,5 und 0,001 %.

Die Herstellung und die Verwendung der erfindungsgemäßen Wirkstoffe geht aus den nachfolgenden Beispielen hervor.

## Herstellungsbeispiele

### Beispiel 1

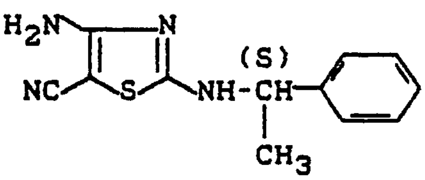

4,2 g (0,10 Mol) Cyanamid werden zu einer auf -10 °C gekühlten und stark gerührten Mischung aus 12,3 g (0,11 Mol) Kalium-tert-butylat und 200 ml Tetrahydrofuran gegeben. Man läßt das Reaktionsgemisch auf 0 °C kommen und gibt bei dieser Temperatur 16,3 g (0,10 Mol) (S)-1-Phenyl-ethyl-isothiocyanat tropfenweise dazu. Anschließend läßt man das Reaktionsgemisch auf Raumtemperatur (ca. 20 °C) kommen und gibt 7,6 g (0,10 Mol) Chloracetonitril dazu. Nach ca. 15-stündigem Rühren wird das Lösungsmittel am Rotationsverdampfer abdestilliert, der Rückstand in Methylenchlorid aufgenommen und mit Wasser geschüttelt. Das dabei kristallin anfallende Produkt wird durch Absaugen isoliert.

Man erhält 17,0 g (70 % der Theorie) (S)-4-Amino-5-cyano-2-(1-phenyl-ethylamino)-thiazol vom Schmelzpunkt 150 °C.

### Beispiel 2

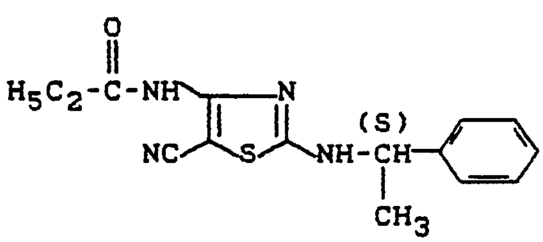

Eine Lösung von 2,0 g (0,022 Mol) Propionsäurechlorid in 10 ml Methylenchlorid wird bei 15 °C unter Rühren zu einer Mischung aus 4,8 g (0,02 Mol) (S)-4-Amino-5-cyano-2-(1-phenyl-ethylamino)-thiazol - Herstellung vgl. Beispiel 1 - 2,3 g (0,03 Mol) Pyridin und 100 ml Methylenchlorid tropfenweise gegeben. Nach 15-stündigem Rühren wäscht man mit 2N-Salzsäure und anschließend mit Wasser, trocknet mit Natriumsulfat und filtriert. Vom Filtrat wird das Lösungsmittel unter Wasserstrahlvakuum sorgfältig abdestilliert, wobei das Produkt als fester Rückstand verbleibt.

Man erhält 5,0 g (83 % der Theorie) (S)-5-Cyano-2-(1-phenyl-ethylamino)-4-propionylamino-thiazol vom Schmelzpunkt 75 °C.

Analog Beispiel 1 und 2 und gemäß der allgemeinen Beschreibung des erfindungsgemäßen Verfahrens können die in der nachstehenden Tabelle 2 aufgeführten Verbindungen der allgemeinen Formel (I) hergestellt werden:

Tabelle 2: Herstellungsbeispiele für die Verbindungen der Formel (I)

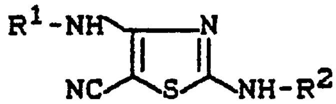

(I)

| Bsp.-Nr. | $R^1$ | $R^2$ | Schmelzpunkt (°C) |
|---|---|---|---|
| 3 | $-CO-CH_3$ | 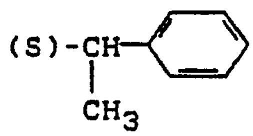 | 82 |
| 4 | H | 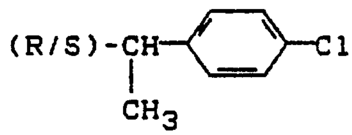 | 210 |
| 5 | $-CO-C_2H_5$ | 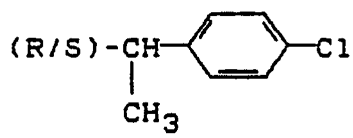 | 170 |
| 6 | $-CO-C_2H_5$ | 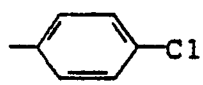 | 68-70 |
| 7 | H | $-(CH_2)_3-CH_3$ | 105 |
| 8 | $-CO-C_2H_5$ | 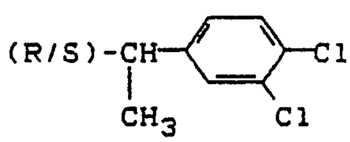 | 183 |
| 9 | $-CO-C_2H_5$ | $-(CH_2)_3-CH_3$ | 164-166 |
| 10 | H | 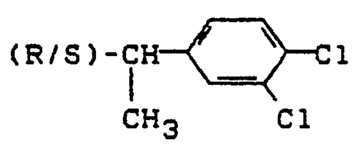 | 198 |

Tabelle 2 - Fortsetzung

| Bsp.-Nr. | $R^1$ | $R^2$ | Schmelzpunkt (°C) |
|---|---|---|---|
| 11 | H | 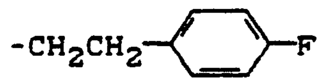 | 165 |
| 12 | H | 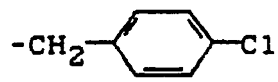 | 211 |
| 13 | H | 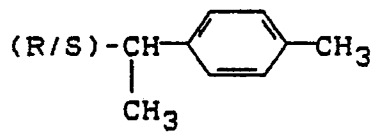 | 196 |
| 14 | H | 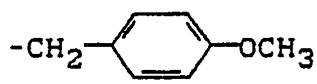 | 211 |
| 15 | $-CO-C_2H_5$ | 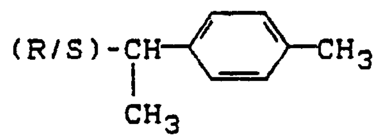 | 60 |
| 16 | H | 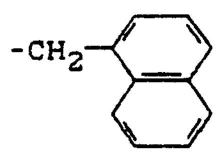 | 217 |
| 17 | $-CO-C_2H_5$ | 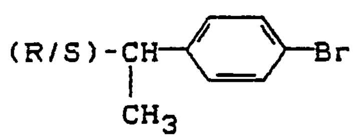 | 170 |
| 18 | $-CO-CH_2OC_2H_5$ | 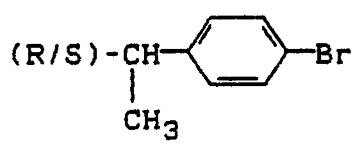 | ($n_D^{20}$ = 1,6085) |

Tabelle 2 - Fortsetzung

| Bsp.-Nr. | $R^1$ | $R^2$ | Schmelzpunkt (°C) |
|---|---|---|---|
| 19 | $-CO-CH_3$ | (R/S)- 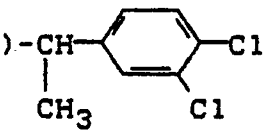 | 240 |
| 20 | $-CO-C_2H_5$ | $-CH_2CH_2$ 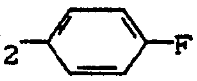 | 172 |
| 21 | $-CO-C_2H_5$ | $-CH_2$ 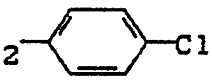 | 226 |
| 22 | H | 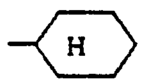 | 160 |
| 23 | $-CO-CH_2CH_2Cl$ | $-CH_2CH_2$ 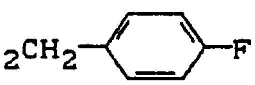 | 173 |
| 24 | $-CO-CH_2CH_2Cl$ | $-(CH_2)_3CH_3$ | 156 |
| 25 | $-CO-C_2H_5$ | $-CH_2$ 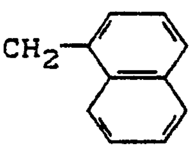 | 200 |
| 26 | $-CO-CH_3$ | $-CH_2$ 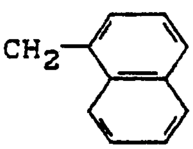 | 180 |
| 27 | H | (R/S)- 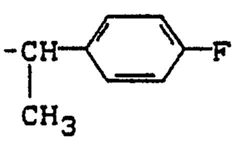 | 189 |

Tabelle 2 - Fortsetzung

| Bsp.-Nr. | $R^1$ | $R^2$ | Schmelzpunkt (°C) |
|---|---|---|---|
| 28 | $-CO-CH_2CH_2OCH_3$ | 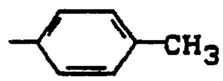 | 174 |
| 29 | H | (R/S)- 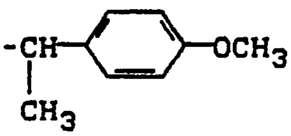 | 192 |
| 30 | $-CO-C_2H_5$ | (R/S)- 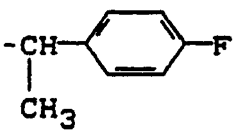 | 109 |
| 31 | $-CO-C_2H_5$ | 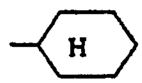 | 182 |
| 32 | $-CO-CH_3$ | 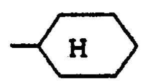 | 181 |
| 33 | $-CO-CH_3$ | (R/S)- 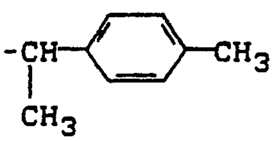 | 172 |
| 34 | $-CO-CH_2CH_2Cl$ | (R/S)- 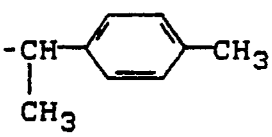 | 120 |
| 35 | $-CO-CH_2OC_2H_5$ | (R/S)- 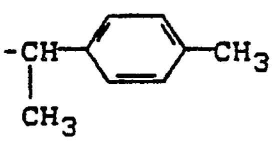 | 130 |
| 36 | $-CO-CH_3$ | (R/S)- 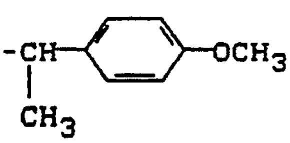 | 192 |

Tabelle 2 - Fortsetzung

| Bsp.-Nr. | $R^1$ | $R^2$ | Schmelzpunkt (°C) |
|---|---|---|---|
| 37 | $-CO-C_2H_5$ | (R/S)- 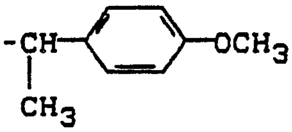 | 131 |
| 38 | $-CO-CH_2OC_2H_5$ | (R/S)- 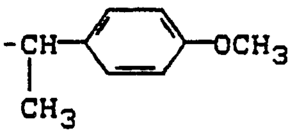 | 88 |
| 39 | $-CO-CH_2CH_2OCH_3$ | (R/S)- 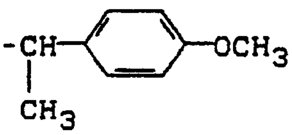 | 110 |
| 40 | $-CO-CH_3$ | (R/S)- 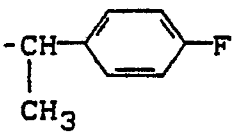 | 166 |
| 41 | $-CO-CH_2OC_2H_5$ | (R/S)- 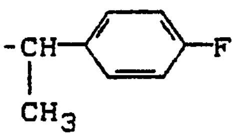 | 92 |
| 42 | $-CO-CH_2CH_2OCH_3$ | (R/S)- 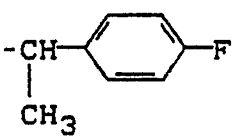 | 115 |
| 43 | $-CO-CH_2CH_2Cl$ | (S)- 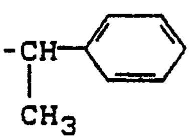 | 77 |
| 44 | $-CO-CH_2OC_2H_5$ | (S)- 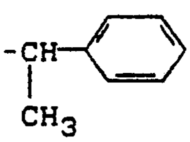 | 118 |

Tabelle 2 - Fortsetzung

| Bsp.-Nr. | $R^1$ | $R^2$ | Schmelzpunkt (°C) |
|---|---|---|---|
| 45 | $-CO-CH_2CH_2OCH_3$ | 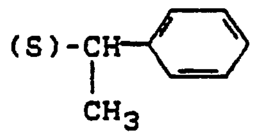 | 131 |
| 46 | H | 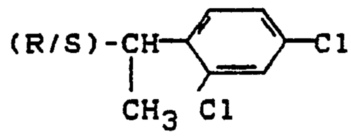 | 216 |
| 47 | H | 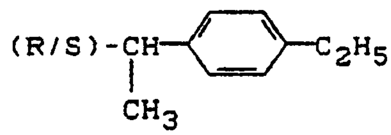 | 178 |
| 48 | H | 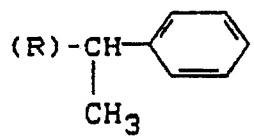 | 187 |
| 49 | $-CO-CH_3$ | 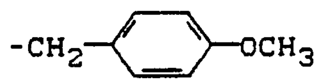 | 231 |
| 50 | $-CO-C_2H_5$ | 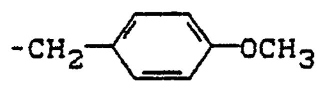 | 184 |
| 51 | $-CO-CH_2OC_2H_5$ | 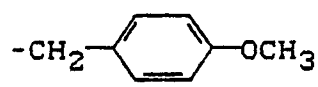 | 80 |
| 52 | $-CO-CH_2CH_2OCH_3$ | 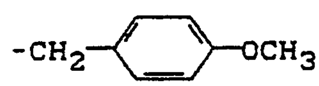 | 128 |
| 53 | $-CO-CH_2CH_2Cl$ | 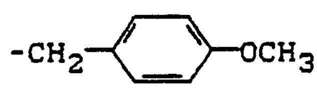 | 182 |

Tabelle 2 - Fortsetzung

| Bsp.-Nr. | $R^1$ | $R^2$ | Schmelzpunkt (°C) |
|---|---|---|---|
| 54 | $-CO-C_2H_5$ | 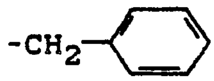 | 185 |
| 55 | $-CO-CH_3$ | 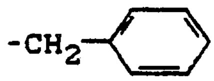 | 216 |
| 56 | $-CO-CH_2CH_2Cl$ | 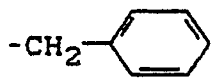 | 195 |
| 57 | $-CO-CH_2OC_2H_5$ | 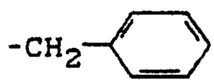 | 128 |
| 58 | $-CO-CH_2OC_2H_5$ | 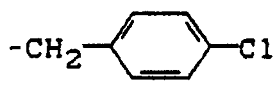 | 148 |
| 59 | $-CO-CH_3$ | 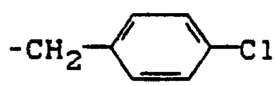 | 265 |
| 60 | $-CO-CH_3$ | 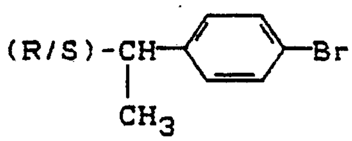 | 158 |
| 61 | $-CO-CH_2CH_2OCH_3$ | 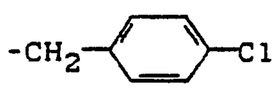 | 178 |
| 62 | $-CO-C_2H_5$ | 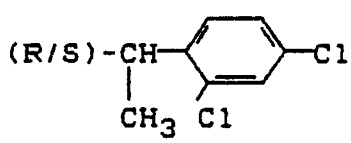 | 198 |
| 63 | $-CO-C_2H_5$ | 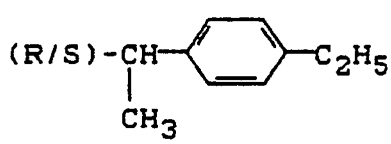 | 198 |

Tabelle 2 - Fortsetzung

| Bsp.-Nr. | $R^1$ | $R^2$ | Schmelzpunkt (°C) |
|---|---|---|---|
| 64 | $-CO-C_2H_5$ | (R)- 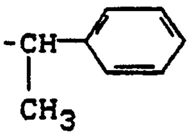 | 159 |
| 65 | $-CO-CH_2CH_2OCH_3$ | (R/S)- 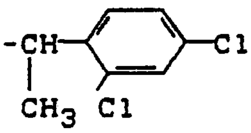 | 176 |
| 66 | $-CO-CH_2CH_2OCH_3$ | (R/S)- 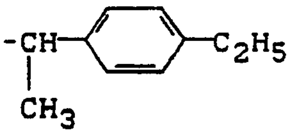 | 88 |
| 67 | $-CO-CH_3$ | (R/S)- 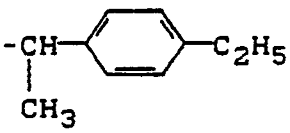 | 80 |
| 68 | $-CO-CH_2OC_2H_5$ | (R/S)- 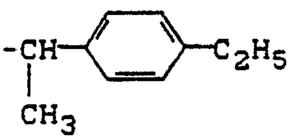 | 110 |
| 69 | H | $C_2H_5$ | 105 |

Verwendungsbeispiele:

In den nachstehenden Verwendungsbeispielen werden die folgenden Verbindungen als Vergleichssubstanzen herangezogen:

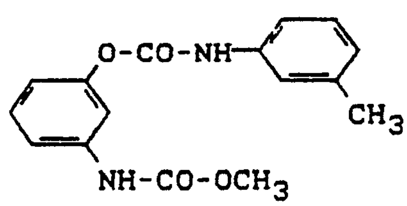

(A)

N-(3-Methyl-phenyl)-O-(3-methoxy-carbonylamino-phenyl)-carbaminsäureester (Phenmedipham) - bekannt aus DE-AS 15 67 151.

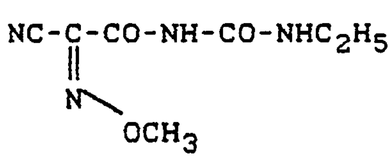

(B)

2-Cyano-N-(ethylamino-carbonyl)-2-methoxyimino-acetamid (Cymoxanil) - bekannt aus US-P 3 957 847.

Beispiel A

Post-emergence-Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausgebracht werden. Nach drei Wochen wird der Schädigungsgrad der Pflanzen bonitiert in % Schädigung im Vergleich zur Entwicklung der unbehandelten Kontrolle. Es bedeuten:
0 % = keine Wirkung (wie unbehandelte Kontrolle)
100 % = totale Vernichtung

In diesem Test zeigen beispielsweise die gemäß den Herstellungsbeispielen (2) und (3) erhaltenen Verbindungen bei guter Verträglichkeit für Kulturpflanzen, wie z. B. Weizen, bei der Bekämpfung von Unkräutern, wie z. B. Amaranthus, Helianthus, Ipomoea, Matricaria und Stellaria, deutlich stärkere Wirkung als die Vergleichssubstanz (A).

Beispiel B

Phytophthora-Test (Tomate) / kurativ

Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit einer wäßrigen Sporensuspension von Phytophthora infestans inokuliert. Die Pflanzen verbleiben 7 Stunden bei 20 °C und 100 % relativer Luftfeuchtigkeit in einer Inkubationskabine. Nach einer kurzen Abtrocknungszeit werden die Pflanzen mit der Wirkstoffzubereitung tropfnaß gespritzt.

Die Pflanzen werden in einer Inkubationskabine mit 100% relativer Luftfeuchtigkeit und ca. 20 °C aufgestellt.

3 Tage nach der Inokulation erfolgt die Auswertung.

Eine deutliche Überlegenheit in der Wirksamkeit gegenüber der aus dem Stand der Technik bekannten Verbindung (B) zeigen in diesem Test z. B. die Verbindungen gemäß den Herstellungsbeispielen (1), (2) und (3).

## Ansprüche

1. 5-Cyano-2,4-diamino-thiazol-Derivate der allgemeinen Formel (I)

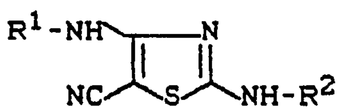

(I)

in welcher
$R^1$ für Wasserstoff oder für einen gegebenenfalls substituierten Rest aus der Reihe Alkyl, Alkenyl oder Alkinyl steht oder für die Gruppierung -CO-$R^3$ steht, worin,
$R^3$ für einen gegebenenfalls substituierten Rest aus der Reihe Alkyl, Alkoxy, Alkylthio, Alkylamino und Dialkylamino steht, und
$R^2$ für einen gegebenenfalls substituierten Rest aus der Reihe Alkyl, Alkenyl, Cycloalkyl, Aryl, Aralkyl, Heteroaryl und Heteroarylalkyl steht,
sowie deren optisch aktive Isomere und
wobei 2-Allylamino-4-amino-5-cyano-thiazol und 2-Phenylamino-4-amino-5-cyano-thiazol ausgenommen sind.

2. 5-Cyano-2,4-diamino-thiazol-Derivate der Formel (I) gemäß Anspruch 1, in welcher
$R^1$ für Wasserstoff, für gegebenenfalls durch Halogen, Cyano, $C_1$-$C_4$-Alkoxy oder $C_1$-$C_4$-Alkoxy-carbonyl substituiertes $C_1$-$C_6$-Alkyl, für gegebenenfalls durch Halogen, Cyano oder $C_1$-$C_4$-Alkoxy-carbonyl substituiertes $C_2$-$C_6$-Alkenyl, für gegebenenfalls durch Halogen, Cyano oder $C_1$-$C_4$-Alkoxy-carbonyl substituiertes $C_2$-$C_6$-Alkinyl oder für die Gruppierung -CO-$R^3$ steht, worin
$R^3$ für einen gegebenenfalls durch Halogen, Cyano, $C_1$-$C_4$-Alkoxy, $C_3$-$C_6$-Cycloalkyl, $C_1$-$C_4$-Alkoxy-carbonyl oder Phenyl substituierten Rest aus der Reihe $C_1$-$C_6$-Alkyl, $C_1$-$C_6$-Alkoxy, $C_1$-$C_6$-Alkylthio, $C_1$-$C_6$-Alkylamino und Di-($C_1$-$C_4$-alkyl)-amino steht, und
$R^2$ für gegebenenfalls durch Halogen, Cyano, $C_1$-$C_4$-Alkoxy, $C_3$-$C_6$-Cycloalkyl oder $C_1$-$C_4$-Alkoxycarbonyl substituiertes $C_1$-$C_6$-Alkyl, für $C_3$-$C_6$-Cycloalkyl, für gegebenenfalls durch Halogen, Cyano oder $C_1$-$C_4$-Alkoxy-carbonyl substituiertes $C_2$-$C_6$-Alkenyl, für gegebenenfalls durch Halogen, Cyano, Nitro, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkylsulfinyl, $C_1$-$C_2$-Halogenalkyl sulfinyl, $C_1$-$C_4$-Alkylsulfonyl, $C_1$-$C_2$-Halogenalkylsulfonyl, $C_1$-$C_2$-Alkylendioxy und/oder $C_1$-$C_4$-Alkoxy-carbonyl substituierte Reste aus der Reihe Phenyl, Naphthyl, Pyridyl, Chinolinyl, Isochinolinyl, Pyrimidinyl, Furyl oder Thienyl steht, oder für gegebenenfalls durch Halogen, Cyano, Nitro, $C_1$-$C_4$-Alkyl, $C_1$-$C_2$-Halogenalkyl, $C_1$-$C_4$-Alkoxy, $C_1$-$C_2$-Halogenalkoxy, $C_1$-$C_4$-Alkylthio, $C_1$-$C_2$-Halogenalkylthio, $C_1$-$C_4$-Alkylsulfinyl, $C_1$-$C_2$-Halogenalkylsulfinyl, $C_1$-$C_4$-Alkylsulfonyl, $C_1$-$C_2$-Halogenalkylsulfonyl und/oder $C_1$-$C_2$-Alkylendioxy substituierte Reste aus der Reihe Phenyl-$C_1$-$C_4$-alkyl, Naphthyl-$C_1$-$C_4$-alkyl, Pyridyl-$C_1$-$C_4$-alkyl, Pyrimidinyl-$C_1$-$C_4$-alkyl, Furyl-$C_1$-$C_4$-alkyl oder Thienyl-$C_1$-$C_4$-alkyl steht,
wobei 2-Allylamino-4-amino-5-cyano-thiazol und 2-Phenylamino-4-amino-5-cyano-thiazol ausgenommen sind.

3. 5-Cyano-2,4-diamino-thiazol-Derivate der Formel (I) gemäß Anspruch 1, in welcher
$R^1$ für Wasserstoff, $C_1$-$C_4$-Alkyl, $C_3$-$C_4$-Alkenyl, $C_3$-$C_4$-Alkinyl oder für die Gruppierung -CO-$R^3$ steht, worin
$R^3$ für gegebenenfalls durch Fluor, Chlor und/oder Brom substituiertes $C_1$-$C_4$-Alkyl, $C_1$-$C_2$-Alkoxy-$C_1$-$C_2$-alkyl, $C_1$-$C_4$-Alkoxy oder $C_1$-$C_4$-Alkylthio steht, und
$R^2$ für $C_1$-$C_5$-Alkyl, für Cyclohexyl, für durch Fluor, Chlor, Brom, Methyl, Ethyl, Trifluormethyl, Methoxy, Ethoxy, Difluormethoxy, Trifluormethoxy, Methylthio, Ethylthio und/oder Trifluormethylthio substituiertes Phenyl, für gegebenenfalls durch Fluor, Chlor, Brom, Methyl, Ethyl, Trifluormethyl, Methoxy, Ethoxy, Difluormethoxy, Trifluormethoxy und/oder Methylendioxy substituiertes Phenyl-$C_1$-$C_3$-alkyl, für Naphthyl-$C_1$-$C_3$-alkyl oder für gegebenenfalls durch Fluor, Chlor, Brom, Methyl, Ethyl, Trifluormethyl, Methoxy und/oder Ethoxy substituiertes Pyridinyl-$C_1$-$C_3$-alkyl steht.

4. Verfahren zur Herstellung von 5-Cyano-2,4-diaminothiazol-Derivaten der allgemeinen Formel (I)

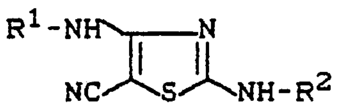

(I)

in welcher
$R^1$ für Wasserstoff oder für einen gegebenenfalls substituierten Rest aus der Reihe Alkyl, Alkenyl oder Alkinyl steht oder für die Gruppierung -CO-$R^3$ steht, worin,
$R^3$ für einen gegebenenfalls substituierten Rest aus der Reihe Alkyl, Alkoxy, Alkylthio, Alkylamino und Dialkylamino steht, und
$R^2$ für einen gegebenenfalls substituierten Rest aus der Reihe Alkyl, Alkenyl, Cycloalkyl, Aryl, Aralkyl, Heteroaryl und Heteroarylalkyl steht,
wobei 2-Allylamino-4-amino-5-cyano-thiazol und 2-Phenylamino-4-amino-5-cyano-thiazol ausgenommen sind,

dadurch gekennzeichnet, daß man Isothiocyanate der allgemeinen Formel (II)

$R^2 - N = C = S$ (II)

in welcher

$R^2$ die oben angegebene Bedeutung hat,

mit Cyanamid in Gegenwart eines Alkalimetallhydroxides oder eines Alkalimetallalkoholates bzw. mit einem Alkalimetallsalz von Cyanamid

und anschließend mit Bromacetonitril oder Chloracetonitril

gegebenenfalls in Gegenwart eines Verdünnungsmittels umsetzt und die so erhaltenen Verbindungen der Formel (I), in welcher dann $R^1$ für Wasserstoff steht - gegebenenfalls nach Zwischenisolierung - gegebenenfalls mit Verbindungen der Formel (III)

$R^1 - X$ (III)

in welcher

$R^1$ mit Ausnahme von Wasserstoff die oben angegebene Bedeutung hat und

X für Halogen steht,

gegebenenfalls in Gegenwart eines Säureakzeptors und gegebenenfalls in Gegenwart eines Verdünnungsmittels umsetzt.

5. Herbizide und fungizide Mittel, gekennzeichnet durch einen Gehalt an mindestens einem 5-Cyano-2,4-diamino-thiazol-Derivat der Formel (Ia)

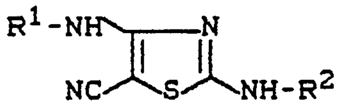

(Ia)

in welcher

$R^1$ für Wasserstoff oder für einen gegebenenfalls substituierten Rest aus der Reihe Alkyl, Alkenyl, Alkinyl steht oder für die Gruppierung -CO-$R^3$ steht, worin,

$R^3$ für einen gegebenenfalls substituierten Rest aus der Reihe Alkyl, Alkoxy, Alkylthio, Alkylamino und Dialkylamino steht, und

$R^2$ für einen gegebenenfalls substituierten Rest aus der Reihe Alkyl, Alkenyl, Cycloalkyl, Aryl, Aralkyl, Heteroaryl und Heteroarylalkyl steht.

6. Verfahren zur Bekämpfung von Unkräutern oder Pilzen, dadurch gekennzeichnet, daß man 5-Cyano-2,4-diamino-thiazol-Derivate der Formel (Ia) gemäß Anspruch 5, auf die Unkräuter oder Pilze oder ihren Lebensraum einwirken läßt.

7. Verwendung von 5-Cyano-2,4-diamino-thiazol-Derivaten der Formel (Ia) gemäß Anspruch 5, zur Bekämpfung von Unkräutern oder Pilzen.

8. Verfahren zur Herstellung von herbiziden oder fungiziden Mitteln, dadurch gekennzeichnet, daß man 5-Cyano-2,4-diamino-thiazol-Derivate der Formel (Ia) gemäß Anspruch 5, mit Streckmitteln und/oder oberflächenaktiven Substanzen vermischt.

Europäisches Patentamt

# EUROPÄISCHER RECHERCHENBERICHT

Nummer der Anmeldung

EP 89 10 1843

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.4) |
|---|---|---|---|
| X | DD-A- 152 937 (K. GEWALD et al.) * Seite 2, Formel III; Seite 5, Zeilen 1,2 * --- | 1-3 | C 07 D 277/56<br>A 01 N 43/78 |
| A | DE-A-3 038 608 (BAYER AG) * Ansprüche 1,10-13 * --- | 1-3,5-8 | |
| A | US-A-3 503 988 (R. LALIBERTE) * Zusammenfassung * --- | 1-3,5,7 | |
| D,A | MONATSHEFTE FUER CHEMIE Band 112, 1981, Seiten 1393-1404; K. GEWALD et al.: "Zur Chemie der 4-Aminothiazolin-2-thione" * Seite 1396, Verbindungen 11A,11B * --- | 1-3 | |
| A | DE-A-2 713 573 (BASF AG) * Seite 4, Formel II; Seite 8, letzter Absatz - Seite 9, Zeile 9 * --- | 1-3 | |
| A | US-A-4 324 899 (M.D. FRISHBERG) * Anspruch 1 * ----- | 1 | **RECHERCHIERTE SACHGEBIETE (Int. Cl.4)**<br>A 01 N 43/00<br>C 07 D 277/00 |

Der vorliegende Recherchenbericht wurde für alle Patentansprüche erstellt

| Recherchenort | Abschlußdatum der Recherche | Prüfer |
|---|---|---|
| BERLIN | 27-04-1989 | HASS C V F |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)